Europäisches Patentamt

European Patent Office

Office européen des brevets

(11) Publication number: **0 142 299**
**B1**

(12) **EUROPEAN PATENT SPECIFICATION**

(45) Date of publication of patent specification: **19.12.90**

(21) Application number: **84307289.3**

(22) Date of filing: **23.10.84**

(51) Int. Cl.⁵: **C 12 Q 1/68**

(54) **Method of measuring polynucleotide and reagent kit for use therein.**

(30) Priority: **25.10.83 JP 199702/83**

(43) Date of publication of application:
**22.05.85 Bulletin 85/21**

(45) Publication of the grant of the patent:
**19.12.90 Bulletin 90/51**

(84) Designated Contracting States:
**CH DE FR GB IT LI NL**

(56) References cited:
**EP-A-0 070 687**
**EP-A-0 123 513**
**WO-A-83/01459**
**US-A-4 302 204**
**US-A-4 351 901**
**US-A-4 358 535**

(73) Proprietor: **FUJIREBIO KABUSHIKI KAISHA also trading as FUJIREBIO INC.**
**6-7, Shimoochiai 4-chome Shinjuku-ku**
**Tokyo 161 (JP)**

(72) Inventor: **Ashihara, Yoshihiro**
**2-402, Fuchudanchi 28-1, Harumicho, 1-chome**
**Fuchu-shi Tokyo (JP)**
Inventor: **Kasahara, Yasushi**
**310, 4-4, Nagayama 3-chome**
**Tama-shi Tokyo (JP)**

(74) Representative: **Silverman, Warren et al**
**HASELTINE LAKE & CO. Hazlitt House 28**
**Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a method of measuring polynucleotide, i.e. deoxyribonucleic acid (DNA) or ribonucleic acid (RNA), in a fluid sample, in particular a body fluid.

The measurement of a DNA in a sample of human serum provides significant results when testing for a viral infection or a hereditary disease. A conventional method of detecting a particular DNA comprises denaturing of a double-stranded DNA (d-DNA) in a sample under investigation to produce a single-stranded DNA (s-DNA), immobilising the s-DNA on a carrier material, hybridising the immobilised s-DNA with a radioisotope-labelled s-DNA, removing unreacted radioisotope-labelled s-DNA, and then measuring radioactivity of the immobilised material. Since this method comprises many processes including immobilisation of each sample, it is complicated to carry out and hence the procedure is time consuming. A procedure of this type is described in EP—A—70687.

It is an object of this invention to provide a simpler method of measuring a polynucleotide.

According to this invention, there is provided a method of measuring a polynucleotide which is either single stranded as such or a single stranded polynucleotide obtained from a double stranded polynucleotide to be measured, which comprises, contacting the single-stranded polynucleotide to be measured with an immobilised labelled single-stranded polynucleotide which is able to hybridise with said single-stranded polynucleotide in aqueous solution to produce a double-stranded polynucleotide, breaking the double-stranded polynucleotide thereby obtained by means of a double-stranded polynucleotide restriction enzyme, and measuring the amount of labelling substance in the solution obtained and/or on immobilised material, as appropriate as a measure of said single stranded and/or said double stranded polynucleotide to be measured.

This invention also provides a reagent kit for use in said method comprising the aforesaid immobilised labelled single stranded polynucleotide and a restriction enzyme.

The method of this invention uses as starting material a single-stranded polynucleotide, hereinafter termed s-polynucleotide to be measured, which may be derived from a double stranded polynucleotide in which case its quantity is a measure of the polynucleotide from which it has been obtained. The s-polynucleotide to be measured may be s-DNA or s-RNA. When the fundamental polynucleotide in a sample to be determined is double-stranded, it is necessary that the polynucleotide be denatured by alkaline treatment using a sodium hydroxide solution or heating to convert it to single-stranded polynucleotide. Test materials which may be subjected to this method include human serum, urine and tissue extracts. When a protein is combined with the polynucleotide in a sample, such as a serum, the protein is preferably separated off by using, for example, a protease.

The immobilised single-stranded polynucleotide (labelled s-polynucleotide) incorporates or has bound thereto a labelling substance and is able to hybridise with the s-polynucleotide to be measured. Hence, this labelled s-polynucleotide acts as a probe which can hybridise with the s-polynucleotide to be measured. The s-polynucleotide to be labelled may be produced by denaturation of a double-stranded polynucleotide containing the s-polynucleotide to be measured by means of alkaline treatment or heating. It may also be produced by a known solid phase polynucleotide synthesis method or by a genetic synthesis method accomplished with γ-DNA using a plasmid. Some s-polynucleotides have, in themselves, found commercial use.

The labelling substance which is used may be a radioisotope, a fluorescent material, a luminescent material, a magnetic material, an enzyme, a prosthetic group of such an enzyme, a coenzyme, or an enzyme inhibitor or activator. Radioisotopes which may be used include $^{32}$P, $^{3}$H, $^{35}$S, $^{14}$C and $^{125}$I. Fluorescent materials which may be used include fluorescent reagents, such as rhodamine, fluorescein, methylcoumarin and dansyl chloride. Luminescent materials which may be used include chemically luminescent materials such as isoluminol and luminol, and a biologically luminescent material such as luciferine-luciferase. Enzymes, for example glucose oxidase, peroxidase and alkaline phosphates, whose activities can easily be measured are preferably used. Since processes for measuring a polynucleotide include heating, the enzyme is preferably thermostable. Should an enzyme which is not thermostable be used, hybridisation may also be carried out at 35 to 40°C. Prosthetic groups include flavine adenine dinucleotide which is an active site of a glucose oxidse, and its amount can be determined by measuring the activity of a holoenzyme after reaction with an apoenzyme. Coenzymes which may be used, include NADH, NADPH$_2$, aminopyrophosphate, pyridoxal phosphate, ADP and ATP.

The carrier material of the labelled s-polynucleotide may be Sepharose, Sephadex (Registered Trade Marks), cellulose gel, ion-exchange resin, filter paper, nitrocellulose, nylon, polystyrene or polyacrylamide.

Immobilisation may be carried out by following a general immobilisation method or a general polynucleotide synthesis route. In such a procedure, an oligonucleotide is first allowed to bind to a carrier material; and then, the s-polynucleotide is allowed to bind to the oligonucleotide by using a 3'-nucleotidase. An amino group or carboxyl group is introduced to the s-polynucleotide, and then it is allowed to bind to a carrier material having carboxyl groups or amino groups by using a reagent for peptide synthesis such as dicyclohexyl carbodiimide or carbonyldiimidazole. When both the s-polynucleotide and a carrier material have amino groups, they can be combined by using for example cyanuric chloride or glutaraldehyde. When both the materials contain SH groups, they can be combined by using a reductant or a reagent having maleimide group. A physical adsorption method may also be employed for the immobilisation. For example, nitrocellulose is immersed in a solution of the s-polynucleotide for a prescribed time and then washed. Finally, the immobilised product may also be produced by connecting nucleotides successively on a carrier material using a solid phase synthesis method.

Introduction of the labelling substance into the s-polynucleotide may be carried out by following a conventional method. The introduction is usually carried out prior to the immobilisation. However, when the labelling substance is a large molecule like an enzyme, the introduction is preferably carrried out after the immobilisation.

The s-polynucleotide to be measured is contacted with the labelled s-polynucleotide in the solution phase. The contacting time is usually about 0.5 to 40 hours. The temperature of the solution is preferably from 20 to 70°C, and the pH is usually 5 to 9.

The double-stranded polynucleotide restriction enzyme then used (hereinafter abbreviated as "restriction enzyme") is preferably one which is specific for double-stranded polynucleotides, and preferably recognises a short polynucleotide chain. Moreover two or more different restriction enzymes may be employed. The restriction enzyme is usually added to the solution after the hybridisation, although it may be added together with or prior to the s-polynucleotide to be measured.

After the restriction enzyme reaction, the immobilised material is separated from the solution, if necessary, and the residual labelling substance in the solution or of the immobilised material, as appropriate, is measured. The measurement may be carried out by a known method. For example, radioactivity of this material may be determined by using a scintillation counter or a Geiger counter in the case of a radioisotope labelling element.

Characteristically, the method of the invention does not need the immobilisation of the s-polynucleotide to be measured, and is easy to carry out. Instead, standard reagents, and optionally devices, can be made available as a kit. A polynucleotide such as DNA can then be easily detected or determined by using this kit.

The following Examples illustrate this invention:

### Example 1
#### (1) Preparation of HBV—DNA Probe

500 ml of a pool serum from chronic B-type hepatitis patients were centrifuged at 9000 rpm for 15 minutes, and the supernatant was further centrifuged using an ultracentrifuge at 4°C at 100,000 xg for 5 hours to collect HBV particles as a pellet. This pellet was dissolved in 10 ml of 0.01 M tris-HCl buffer solution of pH 7.5 containing 0.1 M NaCl, 1 mM EDTA, 0.1% by weight 2-mercaptoethanol and 0.1% by weight of BSA. 5 ml of the virus solution were stored, and the remaining 5 ml were centrifuged at 100,000 xg for 5 hours to obtain a pellet again.

The pellet was treated with 200 μl of 10 mM tris-HCl 0.1 M NaCl pH 7.5 solution containing 0.5% by weight NP—40, and DNA polymerase was thereby activated. 50 μl of 0.08 M $MgCl_2$ 0.2 M tris buffer solution of pH 7.5 containing 1 mMdATP, 1 mMdTTP, 2.5 $\mu M^{32}PdGTP$ and 2.5 $\mu M^{32}PdCTP$ were added to this solution which was allowed to warm up for 3 hours. Then, the solution was layered over 30% by weight sucrose solution placed in a centrifuge tube, and centrifuged at 50,000 rpm for 3 hours using a SW 65 rotor (made by Beckman Co.) to obtain a pellet. This pellet was treated with a protease, and two extractions from the solution were carried each with phenol. The extracts were combined and placed on 5 to 20% by weight sucrose solution gradient, and centrifuged at 50,000 rpm for 3 hours. 15 $S^{32}PDNA$ fractions were collected, and pooled. 15 $S^{32}PDNA$ was precipitated from the pooled fractions by adding ethanol, and dried to obtain the target HBV—DNA.

#### (2) Preparation of DNA Probe by Nick Translation

1 μg of the above HBV-DNA was added to 100 μl of 50 mM tris-HCl buffer solution of pH 7.5 containing a 5 mM $MgCl_2$, 10 mM 2-mercaptoethanol, 5 μMdTTP, 5 μMdGTP, 5 μM $-^{32}P$-dCTP (relative radioactivity 100—700 Ci/mmol) and 5 μM $-^{32}P$-dATP (relative radioactivity 100—700 Ci/mmol). 100 pg DNase I und 100 pg DNA polymeriase I were added to the solution which was incubated at 15°C for 90 minutes. Extraction of radioactive HBV-D-DNA from the solution was carried out by using phenol, and the extract was purified by using a Sephadex G—50 column.

1 mg of this HBV-d-DNA (1 ml) was mixed with 5 mg of single-stranded M13-phage DNA (2 ml). 8 ml of formamide were added to the mixture which was then boiled for 5 minutes. Subsequently, 2 ml of 0.07 M tris-HCl buffer solution containing 2 M NaCl and 15 mM EDTA (pH 7.5) were added to the solution which was then warmed at 50°C for 4 hours and then at 60°C for 1 hour.

The reaction mixture obtained was separated by gel-filtration using Bio-Gel A50 m, and hybridised DNA and unreacted DNA were removed. The first peak fraction near the void fraction was collected, and NaCl powder was dissolved therein in a concentration of 0.1 M. 100% ethanol in a volume twice that of the solution was added, and the solution obtained allowed to stand at −70°C for 2 hours. Subsequently, the solution was centrifuged at 17,000 xg for 10 minutes, and the precipitates which formed were collected. The precipitates were dissolved in 50 ml of 0.001% Phenol Red solution containing 0.1 N NaOH and 0.25 mM EDTA, and purified by gel-filtration using Bio-Gel A50 to obtain the target HBV-s-DNA.

Meanwhile, 1 µg HBV, 100 pg DNase I and 100 pg DNA polymerase I were added to 100 µl of 50 mM tris-HCl buffer solution of pH 7.5 containing 5 mM MgCl₂, 10 mM 2-mercaptoethanol, 5 µMdTTP, 5 µMdGTP, 5 µMdCTP, 5 µMdATP, 10 µM aminohexyl dATP and 10 µM aminohexyl dCTP and the solution was incubated at 15°C for 90 minutes. Extraction from the solution was carried out by using phenol, and the extract was purified by using Sephadex G—50 column to obtain a modified HBV-d-DNA.

This d-DNA was converted to s-DNA by following the aforementioned procedure.

(3) Preparation of Immobilised Material

A polyuracil nucleotide was allowed to bind to CNBr-activated Sepharose gel beads. The radioactive HBV-s-DNA was added to the gel beads, and the 3'-terminal position of the polyuracil was allowed to bind to 5'-terminal position of the radioactive HBV-DNA by adding RNA ligase. The gel beads were sufficiently washed with 50% dimethylformamide containing 0.2 M NaCl to yield the required immobilised material.

1.0 ml of d-DNA restriction enzyme solution (Bgl II, Ava II, Hae II, Hae III, Hap II, Hinc II — each 1 U/ml, 10 mM tris-HCl, 7 mM MgCl₂, 70 mM NaCl, 7 mM 2-mercaptoethanol, pH 7.5) was added to the immobilised material, and allowed to react at 37°C for 1 hour. Then, the immobilised material was sufficiently washed with the same dimethylformamide solution, and HBV-d-DNA was completely removed.

(4) Measurement of Sample

100 µl of 0.5 N NaOH solution were added to 100 µl of the serum of a HB viral hepatitis patient and stirring at ambient temperature was carried out for 10 minutes. Subsequently, the solution was neutralised by adding 100 µl of 0.5 N HCl, and 200 µl of 200 µg/ml proteinase K solution were then added. The solution was allowed to react at 70°C for 1 hour. The immobilised materials prepared in step (3) were suspended in 1 ml of the solution. The mixture was allowed to stand overnight at 37°C. The mixture was centrifuged, and the supernatant was removed. 1.0 ml of d-DNA restriction enzyme solution (Bgl II, Ava II, Hae II, Hae III, Hap II, Hinc II — each 1 U/ml, 10 mM tris-HCl, 7 mM MgCl₂, 70 mM NaCl, 7 mM 2 mercaptoethanol, pH 7.5) was added, and allowed to react at 37°C for 1 hour. After the reaction, the resulting mixture was centrifuged, and the radioactivity of 500 µl, of the supernatant was measured by using a scintillation counter.

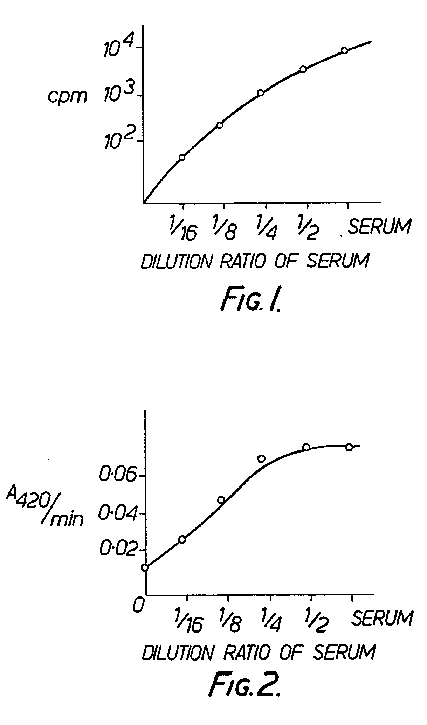

The radioactivity reading was converted to dilution ratio of serum using the previously obtained standard plot of Fig. 1 on which the ordinate axis indicates the counting rate of radioactivity, and the abscissa indicates dilution.

Example 2
(1) Preparation of Immobilised Material

A solution containing 500 µg of aminohexyl-induced HBV-s-DNA was dropped onto a nitro-cellulose filter (5 × 5 cm) so as to permeate all over the filter. the filter was dried by heating under reduced pressure. Subsequently, the filter was washed sufficiently with 0.2 M NaCl, and immersed in 0.1 M carbonate solution of pH 8.0.

100 mg of N⁶-(6-carboxyhexyl)-adenine flavine dinucleotide were dissolved in 10 ml of dimethylformamide. 50 mg of N-hydroxysuccinimide and 70 mg of water-soluble carbodiimide were added to this solution, and reaction was allowed to take place at ambient temperature for 1 hour. After the reaction, this solution was allowed to permeate all over the filter which was allowed to stand at ambient temperature for 2 days. This filter was washed sufficiently with 0.1 M carbonate solution, and, in this way, unreacted aminohexyl adenine flavine dinucleotide was removed.

(2) Measurement

100 µl of 0.5 N NaOH were added to 100 µl of the serum of a HBV viral hepatitis patient, and stirred at ambient temperature of 10 minutes. Subsequently, the solution was neutralised by adding 100 µl of 0.5 N HCl, and 200 µl of 200 µg/ml of proteinase K were then added. The solution was allowed to react at 70°C for 1 hour, and then 200 µl of saturated phenol-chloroform solution (1:1) were added. 200 µl of the aqueous layer separated off therefrom were dropped on to the above filter (1 × 1 cm), and allowed to react at 60°C for 2 hours. 1.0 ml of d-DNA restriction enzyme solution (Hae III, Hinc II, XBa I, Bgl II, Ham HI, Ava II, Alv I — each 1 U/ml, 20 MmM tris-HCl, 7 mM MgCl₂, 70 mM NaCl, 7 mM 2-mercaptoethanol, pH 7.5) was added, and allowed to react at 37°C for 15 minutes. After the reaction, the filter was removed, and 200 µl of a substrate solution (0.025% by weight ABTS, 1.8% β-D-glucose, 7 µg/ml POD, 0.1 M phosphate, pH 6.0) containing 10 µg of apoglucose oxidase were added. Rate assay was carried out by measuring the increase of absorbance at 420 nm.

The reading obtained was converted to dilution ratio of serum using the previously obtained standard plot of Fig. 2 on which the abscissa indicates dilution ratio of serum, and the ordinate inicates absorbance.

**Claims**

1. A method of measuring a polynucleotide which is either single stranded as such or a single stranded polynucleotide obtained from a double stranded polynucleotide to be measured, which comprises, contacting the single-stranded polynucleotide to be measured with an immobilised labelled single-stranded polynucleotide which is able to hybridise with said single-stranded polynucleotide in aqueous solution to produce a double stranded polynucleotide, breaking the double-stranded polynucleotide thereby obtained by means of a double-stranded polynucleotide restriction enzyme, and measuring the amount of labelling substance in the solution obtained and/or on immobilised material, as appropriate as a measure of said single stranded and/or said double stranded polynucleotide to be measured.

2. The method of claim 1, wherein said single-stranded polynucleotide to be measured is a single-stranded deoxyribonucleic acid or a single-stranded ribonucleic acid.

3. The method of claim 1 or 2, wherein said labelling substance is selected from radioisotopes, fluorescent materials, luminescent materials, magnetic materials, enzymes, prosthetic groups thereof, coenzymes and enzyme inhibitors.

4. The method of claim 3, wherein the labelling substance is a large molecule substance and has been introduced into the single-stranded polynucleotide prior to immobilisation thereof.

5. The method of any one of claims 1 to 4, wherein the single-stranded polynucleotide which is labelled has been obtained by denaturation of a double stranded polynucleotide containing the single stranded polynucleotide to be measured.

6. The method of any one of claims 1 to 3, wherein the single-stranded polynucleotide which is labelled has been obtained by a solid phase polynucleotide synthesis method or by genetic synthesis with γ-DNA using a plasmid.

7. The method of any one of the preceding claims, wherein the carrier material is selected from Sepharose, Sephadex, cellulose gel, ion exchange resin, filter paper, nitrocellulose, nylon, polystyrene and polyacrylamide.

8. The method of any one of the preceding claims, wherein said contracting is carried out at from 20 to 70°C at a pH of from 5 to 9 over a period of from 0.5 to 40 hours.

9. The method of any preceding claim, wherein the restriction enzyme is added to the aqueous solution after hybridisation has been effected therein.

10. A kit of reagents for use in the method of any preceding claim which comprises an immobilised labelled single standed polynucleotide which is able to hybridise with a single-stranded polynucleotide in an aqueous solution to produce a double-stranded polynucleotide and a restriction enzyme.

**Patentansprüche**

1. Verfahren zur Messung von Polynukleotiden, die entweder einzelsträngig als solche sind oder als ein einzelsträngiges Polynukleotid aus einem zu messenden doppelsträngigen Polynukleotid erhalten werden, das besteht aus:

dem Zusammenbringen des zu messenden ein-zelsträngigen Polynukleotids mit einem immobili-sierten markierten einzelsträngigen Polynukleotid, das in der Lage ist, mit dem einzelsträngigen Polynukleotid in wässriger Lösung zu hybridisieren, um ein doppelsträngiges Polynukleotid zu produzieren,

dem Brechen des dadurch erhaltenen doppel-strängigen Polynukleotid mittels eines doppel-strängigen Polynukleotidrestriktionsenzymens und

dem Messen der Menge der markierten Substanz in der erhaltenen Lösung und/oder des immobilisierten Materials als zweckdienliches Maß für das zu messende einzelsträngige und/oder doppelsträngige Polynukleotid.

2. Verfahren nach Anspruch 1, bei dem das zu messende einzelsträngige Polynukleotid eine einzelsträngige Desoxyribonukleinsäure oder eine einzelsträngige Ribonukleinsäure ist.

3. Verfahren nach Anspruch 1 oder 2, wobei die Markierungssubstanz ausgewählt wird aus Radio-isotopen, Fluoreszenzmaterialien, Luminiszenz-materialien, magnetischen Materialien, Enzymen, prosthetischen Gruppen davon, Coenzymen und Enzyminhibitoren.

4. Verfahren nach Anspruch 3, wobei die Markierungssubstanz eine hochmolekulare Substanz ist und in das einzelsträngige Polynukleotid eingebracht wird bevor dieses immobilisiert wird.

5. Verfahren nach einem der Ansprüche 1 bis 4, wobei das einzelsträngige Polynukleotid, das markiert ist, erhalten wird durch Denaturierung eines doppelsträngigen Polynukleotids, das das zu messende einzelsträngige Polynukleotid enthält.

6. Verfahren nach einem der Ansprüche 1 bis 3, wobei das einzelsträngige Polynukleotid, das markiert ist, erhalten wird mit einer Festphasen-Poly-nukleotidsynthese-Methode oder durch genet-ische Synthese mit γ-DNA unter Verwendung eines Plasmids.

7. Verfahren nach einem der vorhergehenden Ansprüche, wobei das Trägermaterial ausgewählt wird aus Sepharose, Sephadex, Cellulosegel, Ionenaustauschharz, Filterpapier, Nitrocellulose, Nylon, Polystyrol und Polyacrylamid.

8. Verfahren nach einem der vorhergehenden Ansprüche, wobei das Zusammenbringen bei 20 bis 70°C mit einem pH-Wert von 5 bis 9 in einer Periode von 0,5 bis 40 Stunden durchgeführt wird.

9. Verfahren nach einem der vorhergehenden Ansprüche, wobei das Restriktionsenzym zu der wässrigen Lösung gegeben wird, nachdem die Hybridisierung darin ausgeführt wurde.

10. Reagenzsatz zur Anwendung bei dem Verfahren nach den vorhergehenden Ansprüchen, der besteht aus: einem immobilisierten, markierten einzelsträngigen Polynukleotid, das fähig ist, mit einem einzelsträngigen Polynukleotid in einer wässrigen Lösung zu hybridisieren, um ein dop-pelsträngiges Polynukleotid und ein Restriktions-enzym zu produzieren.

**Revendications**

Procédé pour le dosage d'un polynucléotide qui est soit monocaténaire en tant que tel, soit un polynucléotide monocaténaire obtenu à partir d'un polynucléotide bicaténaire à doser, lequel comprend la mise en contact du polynucléotide monocaténaire à doser avec un polynucléotide monocaténaire marqué, immobilisé, qui est capable d'hybridation avec ledit polynucléotide monocaténaire en solution aqueuse, pour donner un polynucléotide bicaténaire, la coupure du polynucléotide bicaténaire ainsi obtenu, au moyen d'une enzyme de restriction pour polynucléotide bicaténaire, et la mesure de la quantité de substance de marquage dans la solution obtenue et/ou sur le matériau immobilisé, comme convenant en tant que mesure dudit polynucléotide monocaténaire et/ou dudit polynucléotide bicaténaire à doser.

2. Procédé selon la revendication 1, dans lequel ledit polynucléotide monocaténaire à doser est un acide désoxyribonucléique monocaténaire ou un acide ribonucléique monocaténaire.

3. Procédé selon la revendication 1 ou 2, dans lequel ladite substance de marquage est choisie parmi des radioisotopes, des substances fluorescentes, des substances luminescentes, des substances magnétiques, des enzymes, des groupes prosthétiques de celles-ci, des coenzymes et des inhibiteurs d'enzyme.

4. Procédé selon la revendication 3, dans lequel la substance de marquage est une substance macromoléculaire qui a été introduite dans le polynucléotide monocaténaire avant l'immobilisation de celui-ci.

5. Procédé selon l'une quelconque des revendications 1 à 4, dans lequel le polynucléotide monocaténaire qui est marqué a été obtenu par dénaturation d'un polynucléotide bicaténaire contenant le polynucléotide monocaténaire à doser.

6. Procédé selon l'une quelconque des revendications 1 à 3, dans lequel le polynucléotide monocaténaire qui est marqué a été obtenu par une méthode de synthèse de polynucléotide en phase solide ou par synthèse génétique avec de l'ADN-γ, à l'aide d'un plasmide.

7. Procédé selon l'une quelconque des revendications précédentes, dans lequel le matériau support est choisi parmi le Sepharose, le Sephadex, un gel de cellulose, une résine échangeuse d'ions, un papier filtre, la nitrocellulose, le Nylon, le polystyrène et le polyacrylamide.

8. Procédé selon l'une quelconque des revendications précédentes, dans lequel ladite mise en contact est effectuée à une température de 20 à 70°C, à un pH de 5 à 9, pendant une durée de 0,5 à 40 heures.

9. Procédé selon l'une quelconque des revendications précédentes, dans lequel l'enzyme de restriction est ajoutée à la solution aqueuse après que l'hybridation a été effectuée dans celle-ci.

10. Trousse de réactifs à utiliser dans le procédé selon l'une quelconque des revendications précédentes, laquelle comprend un polynucléotide monocaténaire marqué, immobilisé, qui est capable d'hybridation avec un polynucléotide monocaténaire dans une solution aqueuse, pour donner un polynucléotide bicaténaire, et une enzyme de restriction.

FIG. 1.

FIG. 2.